# EUROPEAN PATENT APPLICATION

(11) **EP 3 543 705 A1**
(43) Date of publication of application: **25.09.2019**
(21) Application number: 18163205.0
(22) Date of filing: 21.03.2018
(51) Int. Cl.: G01N 33/84, C07D 209/34, G01N 21/80

(54) **MITOCHONDRIAL PH PROBE**

(71) Applicant: ECOLE POLYTECHNIQUE FEDERALE DE LAUSANNE (EPFL), 1015 Lausanne (CH)
(72) Inventor: The designation of the inventor has not yet been filed
(74) Representative: Gurney, Steven

(57) **Abstract**

The present invention relates to dual fluorophore ratiometric mitochondrial pH probes, their uses and their manufacture.

## Description

### Field of the Invention

The present invention relates to mitochondrial pH probes, their uses and processes to produce them.

### Background to the Invention

Mitochondria are rod-shaped organelles in a cell that convert nutrients into adenosine triphosphate (ATP), using oxygen as an oxidant, thereby providing energy for the cell to drive the cell's metabolic activities. This process is known as aerobic respiration and is the reason animals breathe oxygen. Without mitochondria, cells would only be able to obtain energy from anaerobic respiration which is around 15 times less efficient than aerobic respiration. As well as energy generation, mitochondria regulate cellular redox states, generate most of the cellular reactive oxygen species, and initiate cellular apoptosis.

The number of mitochondria present in a cell depends upon the metabolic requirements of that cell, and may range from a single large mitochondrion to thousands of the organelles. Mitochondria are found in nearly all eukaryotes, including plants, animals, fungi, and protists, and are large enough to be observed with light microscopy.

The specific pH value of the mitochondria directly influences its biological processes and changes to mitochondrial pH can lead to mitochondrial dysfunction. The ability to accurately and sensitively measure mitochondrial pH within living cells can provide important insights into mitochondrial biology, and the dynamics of the living cell as a whole.

A number of fluorescence based mitochondrial pH probes have been developed to measure mitochondrial pH but there remains a need to develop a probe which can rapidly enter the mitochondria and provide reproducible and sensitive measurements without impacting the mitochondrial environment.

### Summary of the Invention

In a first aspect of the present invention there is provided a dual fluorophore ratiometric mitochondrial pH probe of Formula I:

A-L-B (Formula I);

wherein:
A is a pH independent fluorophore;
B is a pH dependent fluorophore; and
L is a chemical linker, conjugating the pH independent fluorophore A to the pH dependent fluorophore B, or L is a covalent bond;
wherein in an inactive form, the probe is protected in an overall positive oxidation state; and wherein the probe may be deprotected intracellularly into an active form with a variable oxidation state.

The present inventors have found that by protecting the pH dependent fluorophore of a dual fluorophore ratiometric probe into a positive oxidation state, the probe is efficiently transported into the mitochondria of the cell and is converted by the cell into an active form where the oxidation state of the pH dependent fluorophore can vary enabling sensitive and rapid evaluation of mitochondrial pH.

In a further aspect there is provided a method of measuring mitochondrial pH comprising the steps of:
a) contacting a cell with a mitochondrial pH probe in an inactive form as described herein;
b) allowing the probe to accumulate in the mitochondria of the cell, and to convert into an active form;
c) determining the ratio of fluorescence between the pH independent fluorophore A and the pH dependent fluorophore B; and
d) using this ratio to determine the mitochondrial pH.

In a yet further aspect there is provided a method of measuring the impact of drugs or other conditions on the mitochondrial pH comprising applying the drug or other condition to a cell and measuring the change in mitochondrial pH using a dual fluorophore ratiometric mitochondrial pH probe as described herein.

In a yet further aspect there is provided a method of manufacturing a dual fluorophore ratiometric mitochondrial pH probe of Formula I:

A-L-B (Formula I);

wherein:
A is a pH independent fluorophore;
B is a pH dependent fluorophore; and
L is a chemical linker, conjugating the pH independent fluorophore A to the pH dependent fluorophore B, or L is a covalent bond;
as described herein, comprising protecting the pH dependent fluorophore B such that the mitochondrial pH probe of Formula I is put into a positive oxidation state.

### Description of the Drawings

Figure 1 is a schematic view of a mitochondria organelle
Figure 2 is a schematic view of a cell showing the membrane barriers, mitochondria and pH probe
Figure 3 is a plot of fluorescein channel pH-dependent fluorescence in PBS-5% BSA buffer for 5-FA-PIP-Cy5 (the unprotected form of the probe, which is active in the intracellular environment)
Figure 4 is a series of images showing typical pH variations in mitochondria of A2780 ovarian cancer cell line are represented. Figure 4A represents the 5FA-PIP-Cy5-DA pH-independent (Cy5) channel. Figure 4B represents the 5FA-PIP-Cy5-DA pH-dependent (5Fluo) channel. Figure 4C represents the overlap image of Figure 4A and Figure 4B. Figure 4D represents the bright field image
Figure 5 is the fluorescein/Cy5 ratio measured in homeostatic conditions and upon nigericin and FCCP treatment
Figure 6 is a plot of the ratio of fluorescein/Cy5 at a range of pH values when the mitochondrial proton gradient is collapsed with FCCP
Figure 7 is a plot of the ratio of fluorescein/Cy5 at a range of pH values in the presence of nigericin
Figure 8 is a series of typical images of mitochondria, stained with both 5FA-PIP-Cy5-DA. Figure 8A represents 5FA-PIP-Cy5-DA pH independent (Cy5) channel, shown in red. Figure 8B represents 5FA-PIP-Cy5-DA pH-dependent (5Fluo) channel, shown in blue. Figure 8C represents TMRM channel, shown in magenta. Figure 8D represents bright field image
Figure 9 is a series of images which show the advantage of the caging approach through the staining of A2780 cells with either 5FA-PIP-Cy5-DA or 5FA-PIP-Cy5 in identical conditions. Figure 9A shows the successful staining of mitochondria in the case of inactive form 5FA-PIP-Cy5-DA. Figure 9B shows unsuccessful staining of mitochondria in the case of active form 5FA-PIP-Cy5. Figure 9C shows negative control cells with no staining agent present
Figure 10 is a plot of a 5FA-PIP-Cy5-DA pH dependence calibration curve, obtained with MPP cells in the presence of FCCP
Figure 11 is a plot to show the estimated mitochondrial pH (single haematopoietic cells) in different phenotypes and culture conditions as measured with 5-FA-PIP-Cy5-DA
Figure 12 is a graph to show the 5FA-PIP-Cy5-DA pH dependence calibration curve obtained with A2780 cells in the presence of FCCP
Figure 13 is a plot of estimated mitochondrial pH (average per field of view) of A2780 cells in the presence of anti-cancer drugs and following the drugs' removal

### Detailed Description of the Invention

In the present application, a number of general terms and phrases are used, which should be interpreted as follows.

"Mitochondrial pH probe" means a molecule which is capable of relaying information about the pH inside a mitochondrial matrix.

"Mitochondria" are rod-shaped organelles in a cell that convert nutrients into adenosine triphosphate (ATP), using oxygen as an oxidant, thereby providing energy for the cell to drive the cell's metabolic activities. Two specialized membranes encircle each mitochondrion present in the cell. The membranes divide the organelle into a narrow intermembrane space and a much larger internal matrix, each of which contains highly specialized proteins. The outer membrane of a mitochondrion contains many channels formed by the proteins like porin and acts like a sieve, filtering out molecules that are too big. Similarly, the inner membrane, which is highly convoluted so that a large number of infoldings called cristae are formed, also allows only certain molecules to pass through it and is much more selective than the outer membrane. To make certain that only those materials essential to the matrix are allowed into it, the inner membrane utilizes a group of transport proteins that will only transport the correct molecules.

Mitochondria are generally oblong organelles, which range in size between 0.5 and 10 micrometers in length, and occur in numbers that directly correlate with the cell's level of metabolic activity. The organelles are quite flexible, however, and time-lapse studies of living cells have demonstrated that mitochondria change shape rapidly and move about in the cell almost constantly. Movements of the organelles appear to be linked in some way to the microtubules present in the cell, and are probably transported along the network with motor proteins. Consequently, mitochondria may be organized into lengthy traveling chains, packed tightly into relatively stable groups, or appear in many other formations based upon the particular needs of the cell and the characteristics of its microtubular network.

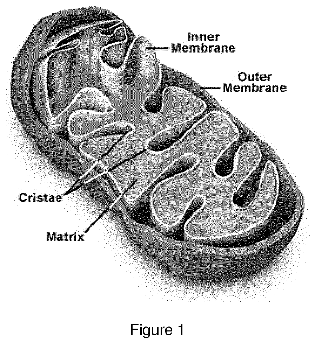

An example of mitochondria is shown in Figure 1 which shows the outer membrane 1, the inner membrane 2 demonstrating the infolding leading to formation of cristae 3, and the mitochondrial matrix 4. The probes of the present invention are able to measure the pH of the matrix 4.

"Fluorophore" means a fluorescent chemical compound that can re-emit light upon light excitation, emitted light being of a different wavelength than excitation light.

"Dual fluorophore" means a molecule that contains two or more covalently linked fluorophores that are either directly connected together or are tethered together by a linker. Upon light excitation, each fluorophore can re-emit light.

"pH independent fluorophore" means a fluorophore which emits light at a specific wavelength largely irrespective of the pH of the environment it is in. A pH independent fluorophore therefore exhibits consistent fluorescence over variable pH conditions, for example both low and high pH.

"pH dependent fluorophore" means a fluorophore whose fluorescence changes depending on the pH of the environment it is in. A pH dependent fluorophore is sensitive to the pH environment and exhibits variable fluorescence depending on pH.

"Ratiometric" means a method of measuring the fluorescence of the flurophores and determining the fluorescence ratio. Applying a ratiometric approach avoids flurophore concentration effects from influencing the pH measurement. If a probe comprises a single pH dependent fluorophore, the intensity of the emission will be dependent not only on pH but also on the fluorophore concentration. This makes it difficult to compare results between different biological samples. By applying a ratiometric approach, internal normalisation of the fluorescence readings is possible. While the absolute fluorescence intensities between different samples may vary depending on the fluorophore concentrations, the fluorescence ratio will remain independent of concentration and will only vary depending on pH.

"Linker" means a sub-unit of a molecule which serves to covalently link two other subunits of said molecule together. In the present invention, the linker may be absent whereby the two units are directly linked to each other, or may be present and provide the link between the units. A linker should not adversely affect the function of the units. A linker should not prevent the individual fluorophores from fluorescing in a pH dependent or pH independent manner as required. For example, a linker should not conjugate into the fluorescence causing region of the flurophores, or should not do so in a way that adversely changes the properties of the flurophore(s).

"Inactive form" means the molecule has been modified such that it exists in a positive oxidation state. In some embodiments, in inactive form, the molecule does not fluoresce in a pH dependent manner. The molecule may be in inactive form via protection of one or more functional groups. When in inactive form, the molecule is configured to facilitate transportation into the target location, for example the cell and/or mitochondria.

"Active form" means the molecule is configured to produce its desired effect e.g. measure mitochondrial pH. In active form, the molecule will fluoresce in a pH dependent manner to enable mitochondrial pH measurement. The molecule may be converted into active form via removal of the protecting group(s).

"Protecting group" means a group which has been introduced into a molecule by modification of a functional group which prevents said functional group from undergoing further changes, (e.g. reactions) until the protecting group has been removed. Suitable protecting groups can be deprotected intracellularly. Suitable protecting groups include alcohol protecting groups, amine protecting groups, carboxylic acid protecting groups, and the like.

Examples of alcohol protecting groups include esters, formed with saturated and aromatic carboxylic acids, organic carbonate esters.

Examples of amine protecting groups include amides, formed with saturated and aromatic carboxylic acids, carbamates, thiocarbamates.

Examples of carboxylic acid protecting groups include acetoxymethyl ester, anhydrides, formed with saturated and aromatic carboxylic acids.

"Oxidation state" means the overall charge of an atom, part of a molecule or a molecule. A single positive charge in a molecule corresponds to an oxidation state of +1 and a single negative charge corresponds to an oxidation state of -1. The oxidation state of a molecule or part of a molecule can be condition dependent or independent. For example, a nitrogen with four alkyl bonds exists in a +1 oxidation state irrespective of the surrounding conditions (e.g. pH). By comparison, an alcohol molecule ROH exemplifies a variable oxidation state as it can exist at multiple oxidation states depending on the pH of its surroundings. At low pH the oxygen will bond to H⁺, leaving the alcohol ROH₂⁺ in a +1 oxidation state. Alternatively, at a high pH, H⁺ may be lost, leading to a molecule in a -1 oxidation state as RO⁻. As a further example a molecule with the fixed oxidation state nitrogen and the variable oxidation state alcohol both present would display a variable oxidation state, moving from +1 in inactive form of the molecule to 0 for the active form of the molecule at low pH to -1 for the active form of the molecule at high pH.

"Converted intracellularly" means a process whereby a molecue is converted from one form to another (e.g. from an inactive form to an active form) via cellular machinery inside a cell. For example, a molecule in protected form may be deprotected intracellularly by esterases to convert it from an inactive form into an active form. A protected molecule may therefore be deprotected intracellular.

"Probe calibration" means the measurement of probe response in known conditions in order to produce quantifiable condition/response data, which can be used in combination with experimental probe data to determine the conditions present during use of the probe.

"Measuring the impact of drugs or other conditions" means using the probes of the present invention to determine the impact of drug(s) or other situations on mitochondrial pH. For example, the probes of the present invention may be used to determine how mitochondrial pH changes in response to the application of a drug into a cellular system and/or the removal of a drug from a cellular system. The probe of the present invention may also be used to determine how mitochondrial pH changes in response to other changes to cellular conditions, for example changes to temperature or the like. The probes of the present invention may be used to monitor real time changes to mitochondrial pH in response to any stimulus or condition being applied to the cell.

The present invention relates to dual fluorophore ratiometric mitochondrial pH probes of general formula I as defined above.

In the molecules of the present invention, the groups can be selected in accordance with the following guidance:

"Alkyl" means groups which may be branched or unbranched, and preferably have from 1 to about 12 carbon atoms. One more preferred class of alkyl groups has from 1 to about 8 carbon atoms. Even more preferred are alkyl groups having 1, 2, 3, 4, 5, 6, 7 or 8 carbon atoms. Propyl including isopropyl, butyl including sec-butyl and isobutyl, pentyl, including isopentyl, hexyl, heptyl and octyl are particularly preferred alkyl groups in the compounds of the present invention. Alkyl groups according to the present invention may be unsubstituted or substituted.

"Alkenyl" and "alkynyl" mean groups which may be branched or unbranched, have one or more unsaturated linkages and from 2 to about 12 carbon atoms. One more preferred class of alkenyl and alkynyl groups has from about 2 to about 8 carbon atoms.. Even more preferred are alkenyl and alkynyl groups having 2, 3, 4, 5, 6, 7 or 8 carbon atoms. Alkenyl and alkynyl groups according to the present invention may be unsubstituted or substituted.

"Cycloalkyl" means a cyclic saturated monovalent hydrocarbon group including single or multiple ring structures, including multiple ring groups that contain separate and/or fused alkyl rings. Typical cycloalkyl groups contain from 1 to 3 separate and/or fused rings and from 3 to about 18 carbon ring atoms. Preferably cycloalkyl groups contain from 6 to about 12 carbon ring atoms. Specially preferred cycloalkyl groups include cyclopropyl, cyclobutyl, cyclopentyl, cyclohexyl, cycloheptyl, bicyclohexane and decalin. Cycloalkyl groups according to the present invention may be unsubstituted or substituted.

"Heteroalkyl" means an alkyl group, as defined above, having a substituent containing at least one heteroatom selected from NR', O, S, where R' may be a suitable group such as H, C₁-C₁₂ alkyl, C₂-C₁₂ alkenyl, C₂-C₁₂ alkynyl, aryl, and heterocyclic group. The R' groups according to the present invention may themselves be unsubstituted or substituted. These heteroatoms may optionally terminate the alkyl chain with a general formula of RNR', RO or RS or may be within the group with a general formula of RNR'R", ROR" or RSR". Preferred heteroalkyls include the following groups (note: when used in a linker the groups have one or more hydrogen atoms removed to form a bond with a flurophore(s) and/or another portion of the linker) ethylamine, propylamine, isopropylamine, butylamine, sec-butylamine, isobutylamine, pentylamine, hexylamine, ethanol, propanol, isopropanol, butanol, sec-butanol, isobutanol, pentanol, hexanol, ethanethiol, propanethiol, isopropanethiol, butanethiol, sec-butanethiol, isobutanethiol, pentanethiol, hexanethiol, methylenediamine, diaminoethane, diaminopropane, diaminobutane, diaminopentane, hexamethylenediamine, ethanolamine, aminomethylpropanol, heptaminol, 6-amino-1-hexanol, 2-aminoethanethiol, 3-aminopropanethiol, 4-aminobutanethiol, 5-aminopentanethiol, 6-aminohexanethiol, dimethylamine, diethylamine, dipropylamine, diisopropylamine, dibutylamine, disecbutylamine, diisobutylamine, dipropylamine, dihexylamine, dimethylether, diethylether, dipropylether, diisopropylether dibutylether, disec-butylether, diisobutylether, dipentylether, dihexylether, dimethylsulfide, diethylsulfide, dipropylsulfide, diisopropylsulfide, dibutylsulfide, disec-butylsulfide, diisobutylsulfide, dipentylsulfide or dihexylsulfide. Heteroalkyl groups according to the present invention may be unsubstituted or substituted.

"Halo" means fluoro (F), chloro (CI), bromo (Br) or iodo (I).

"Aryl" means an aromatic system including single and multiple ring compounds, including multiple ring compounds that contain separate and/or fused aryl groups. Typical aryl groups contain from 1 to 3 separated or fused rings and from 6 to about 18 carbon ring atoms. Preferably aryl groups contain from 6 to about 12 carbon ring atoms. Specially preferred aryl groups include phenyl, naphthyl, biphenyl, unsubstituted phenanthryl and anthryl. Aryl groups according to the present invention may be unsubstituted or substituted.

"Heterocyclic" includes aromatic (heteroaromatic) or saturated or non-aromatic unsaturated (both heteroalicyclic) rings containing from 1 to 3 separated or fused rings and about 5 to 18 ring atoms. Preferably heterocyclic rings contain from 5 to about 10 ring atoms, preferably 5, 6 or 7 ring atoms. Suitable heterocyclic groups in the compounds of the present invention contain one, two or three heteroatoms selected from N, O or S atoms. Suitable heterocyclic groups include 8-quinolyl, isoquinolyl, pyridyl, pyrazinyl, pyrazolyl, pyrimidinyl, furyl, pyrrolyl,thienyl, thiazolyl, isothiazolyl, triazolyl, tetrazolyl, isoxazolyl, oxazolyl,imidazolyl, indolyl, isoindolyl, indazolyl, indolizinyl, phthalazinyl,pteridinyl, purinyl, oxadiazolyl, thiadiazolyl, furazanyl, pyridazinyl,triazinyl, cinnolinyl, benzimidazolyl, benzofuranyl, benzofurazanyl, benzothiophenyl, benzothiazolyl, benzoxazolyl, quinazolinyl,quinoxalinyl, naphthyridinyl, furopyridyl, pyrrolidinyl, tetrahydrofuranyl, dihydrofuranyl, tetrahydrothienyl,tetrahydrothiopyranyl, piperidyl, morpholinyl, thiomorpholinyl, thioxanyl, piperazinyl, azetidinyl, oxetanyl, thietanyl, homopiperidyl, oxepanyl, thiepanyl, oxazepinyl, diazepinyl, thiazepinyl, 1,2,3,6-tetrahydropyridyl, 2-pyrrolinyl, 3-pyrrolinyl, indolinyl, 2H-pyranyl, 4Hpyranyl, dioxanyl, 1,3-dioxolanyl, pyrazolinyl, dithianyl, dithiolanyl,dihydropyranyl, dihydrothienyl, dihydrofuranyl, pyrazolidinyl, imidazolinyl, imidazolidinyl, 3-azabicyclo[3.1.0]hexyl, 3-azabicyclo[4.1.0]heptyl, 3H-indolyl, and quinolizinyl. Heterocyclic groups according to the present invention may be unsubstituted or substituted.

"Alkylcarbonyl", "alkenylcarbonyl", "alkynylcarbonyl" mean an alkyl, alkenyl or alkynyl chain as described above which includes one or more carbonyl groups. These groups may terminate the alkyl chain or may be within its structure. If the carbonyl group terminates the alkyl chain it may optionally be bonded to a heteroatom selected from NR', O, S to form an amide, ester or thioester bond respectively, where R' is defined as above. Preferred alkylcarbonyls, alkenylcarbonyls and alkynylcarbonyls include acetaldehyde, propanal, butanal, isobutanal, pentanal, isovaleraldehyde, hexanal, acetone, butanone, methylisopropylketone, pentan-2-one, pentan-3-one, hexan-2-one, hexan-3-one, glyoxal, malondialdehyde, succinaldehyde, glutaraldehyde, hexanedial, oxobutanal, 4-oxopentanal, 4-oxohexanal, 5-oxohexanal, 2,4-pentanedione, 2,3-hexanedione, 2,4-hexanedione, 2,5-hexanedione, 4-penten-2-one, *trans*-3-pentanal, 3-methyl-3-pentenal, 4-hexen-3-one, 3-butynaldehyde, 3-pentynylaldehyde, 3-hexynylaldehyde, 4-hexynynlaldehyde, 4-hexynylhexan-2-one. Alkylcarbonyls, alkenylcarbonyls and alkynylcarbonyls groups according to the present invention may be unsubstituted or substituted.

The groups above mentioned may be substituted at one or more available positions by one or more suitable groups such as OR', =O, SR', SOR', SO2R', NO2, NHR', NR'R', =N-R', NHCOR', N(COR')2, NHSO₂R', NR'C(=NR')NR'R', CN, halogen, COR', COOR', OCOR', OCONHR', OCONR'R', CONHR', CONR'R', protected OH, C₁-C₁₂ alkyl, C₂-C₁₂ alkenyl, C₂-C₁₂ alkynyl, aryl, and heterocyclic group, wherein each of the R' groups is independently selected from the group consisting of hydrogen, OH, NO₂, NH₂, SH, CN, halogen, COH, COalkyl, CO₂H, C₁-C₁₂ alkyl, C₂-C₁₂ alkenyl, C₂-C₁₂ alkynyl, aryl, and heterocyclic group. The optional substituents described in this paragraph may themselves be substituted. Where such groups are themselves substituted, the substituents may be chosen from the foregoing list.

In an inactive form, the mitochondrial pH probes of the present invention are protected such that they exist in a positive oxidation state. In a preferred embodiment, the probe is in a fixed oxidation state of +1.

By fixing the probe into a positive oxidation state, the probe will preferentially accumulate within the mitochondria. Additionally, in the inactive form the probe is much more lypophilic, thus permeating the cellular membranes much more rapidly. Mitochondria are distinguished from other cellular organelles by its inner mitochondrial membrane having a high electrochemical potential. This electrostatic potential pulls the positively charged probe into the mitochondria leading to a concentration of probe in the mitochondria several orders of magnitude higher than the rest of the cell. The positive charge on the probe also helps with the initial delivery of the probe into the cell since the cell plasma membrane also has an electrochemical potential of the same polarity of the mitochondria, albeit at a much lower magnitude.

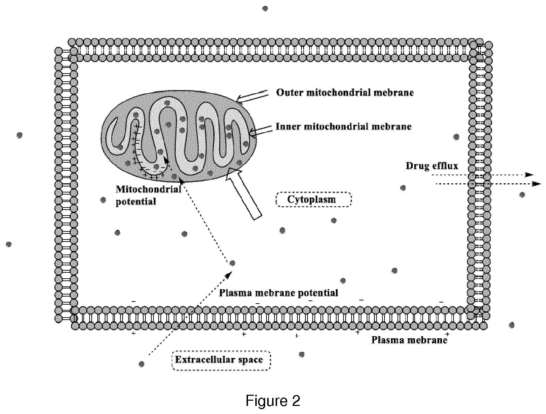

In fact, mitochondrial accumulation is influenced by three main factors as shown in Figure 2, namely:
1. initial accumulation in the cellular cytoplasm, dependent on plasma membrane potential;
2. secondary preferential accumulation in the mitochondria, dependent on the mitochondrial membrane potential; and
3. active cytoplasmic probe efflux by the cell removing the probe (which is highly cell dependent).

Figure 2 shows a schematic representation of a cell (10) including a plasma membrane (11) and cytoplasm (12). The cell includes a mitochondrion organelle (13) which comprises an outer mitochondrial membrane (14) and an inner mitochondrial membrane (15). Mitochondrial pH probe molecules according to the present invention are shown as dots (16). The pH probe is initially outside the cell in extracellular space but the probe travels through the plasma membrane (11). The positive oxidation state of the probe and its suitable lipophilicity encourages influx into the cell.

Once in the cytoplasm (12), the probe is attracted into the mitochondria, again due to its positive oxidation state. The probe then accumulates inside the mitochondria (13) in the mitochondrial matrix. The probe is converted via cellular machinery (for example esterases) into active form. While this conversion will take place within the whole cell, the preferential accumulation into the mitochondria due to the positive probe charge leads to an overall accumulation inside the mitochondria.

The diagram shows drug efflux which has the effect of removing probe from the cell. The relative ratio of probe influx and probe efflux is determined by the difference between the speed of the probe crossing the plasma membrane and the ability of the cell to remove probe by cell defensive mechanisms. Having a probe which can efficiently cross the cell membrane can increase the ratio of accumulation relative to efflux and therefore probes with high lipophilicity are generally preferred.

By protecting the probe, for example by acylating phenolic groups on the pH dependent flurophore, it is possible to increase the lipophilicity of the probe and therefore improve the ability for the probe to travel through both the cell's plasma membrane and also through the inner and outer mitochondrial membranes.

Thus, protecting the probe according to the present invention can improve both the speed at which the probe accumulates within the mitochondria and also the overall concentration of probe contained within the mitochondria relative to the probe contained within the cell's cytoplasm and extracellularly. The probe achieves this by fixing a positive oxidation state to pull the probe into the mitochondria; and/or by increasing the lipophilicity of the probe. Through these mechanisms, the influx of the probe into the mitochondria outweighs the efflux out of the cell.

The mitochondrial pH probe of the present invention is converted intracellularly into an active form by removal of the protecting groups and therefore converting the probe from a positive oxidation state into an active variable oxidation state. Additionally, the active form of the probe is much more hydrophilic in comparison to the inactive form, thus has a very low membrane permeability and gets trapped inside the mitochondrial matrix. Preferably, the oxidation state of the active probe varies between an oxidation state of 0 and -2. More preferably, the active probe has a variable oxidation state between 0 and -1. Alternatively, the active probe has a variable oxidation state between -1 and -2.

Converting the probe into a lower oxidation state when in active form has a number of advantages. Firstly, while the positive oxidation state facilitates transportation into the mitochondria, converting the probe into a neutral or negative oxidation state reduces the possibility that the accumulation of positively charged probe depolarises the mitochondria and therefore negatively impacts the cell and the results obtained. Otherwise, the high accumulation of positively charged probes may influence mitochondrial activity due to depolarisation of the mitochondrial matrix thereby influencing or invalidating the results obtained.

Secondly, having crossed into the mitochondrial matrix, converting into an active form reduces the lipophilicity of the probe and therefore makes it more difficult for the cellular machinery to efflux the probe. For example, removing protection from an otherwise hydrophilic group will significantly increase the hydrophilicity of the probe and therefore greatly reduce lipophilicity. This effectively traps the probe inside the lipophilic membrane. The inner mitochondrial membrane is particularly tight which means that lipophilicity is important to enable the probe to cross the membrane. Once converted into active form, the change in lipophilicity helps to trap the probe inside the inner membrane and in the mitochondrial matrix.

Thus, the mitochondrial pH probes of the present invention are configured to rapidly accumulate in the mitochondria relative to the cell's cytoplasm or extracellularly, and to convert intracellularly into an active form which does not depolarise the mitochondria thereby affecting the function of the mitochondria. This leads to a number of advantages.

The preferential accumulation of probe in the mitochondria enables accurate and sensitive measurements. By having a high accumulation of active probe in the mitochondria, it is possible to sensitively determine the pH of the mitochondrial matrix and the readings are not unduly influenced by probe readings in other parts of the cell or extracellularly. This allows for sensitive readings able to measure small changes in mitochondrial pH.

The rapid accumulation enables fast pH measurements to be taken. It is possible to measure mitochondrial pH in real time and to rapidly follow the impact of external forces, for example drugs, on mitochondrial pH. Through these rapid real time measurements, it is possible to track mitochondrial pH changes and see the influence of drugs or other external factors on cellular mechanisms.

The probes of the present invention are ratiometric. By covalently linking a pH dependent fluorophore with a pH independent fluorophore, it is possible to take concentration independent pH measurements. This allows for results to be normalised and compared between biological samples with variable nature.

With a single fluorophore approach, there is just a single wavelength region of a pH-dependent fluorophore, where fluorescence intensity is measured. Different biological samples can only be compared between each other if the concentrations of the fluorophore in these samples are equal. With the ratiometric probes of the present invention, while the absolute fluorescent intensities of different samples may vary dependent on the corresponding fluorophore concentrations, the fluorescence ratio remains independent of the concentration and only responds to the change in pH.

According to a preferred embodiment, the two fluorophores have spectrally separated fluorescence bands and/or absorption bands. This improves the sensitivity of the readings and allows for more accurate measurements to be taken.

When two emission or excitation spectral bands used for the ratiometric approach correspond to the same single fluorophore, these bands are usually highly overlapping spectrally. This makes the pH-dependent fluorescence ratio change relatively small and thus the probe sensitivity low. While it is possible to use specially designed optical filters to increase probe sensitivity, this requires modification of the microscopes and thus causes impracticality. In contrast, if the two emission/excitation bands are completely spectrally separated, and are within the standard optical filter wavelength range (in this case green (GFP/FITC) and deep red (Cy5) ranges), then the sensitivity of the probe is greatly increased and can be used with any standard imaging system.

The probes of the present invention comprise two fluorophores covalently linked together. Covalently linking fluorophores leads to advantageous properties. Were two non-covalently linked fluorophores to be used, the ratiometric approach would rely on the same concentration of each probe being present in the mitochondria at the same time. In practice, this is difficult to achieve because different probes will influx and efflux at different rates. By covalently linking the fluorophores together, the concentration ratio between them is inherently constant, because it is defined by the stoichiometry of the molecule.

The probe is converted from an inactive protected form into an active deprotected form intracellularly. In a preferred embodiment, esterases within the cell (mostly within mitochondria) deprotect the probe. The probe and the linker are configured so that said intracellular deprotection does not otherwise adversely affect the probe, for example by not only cleaving the protecting groups but also by cleaving other elements of the probe, for example the linker.

The probes according to the present invention may incorporate any suitable fluorophores. The fluorophores may be selected such that they fluoresce or absorb light at spectrally distinct regions. The fluorophores are also selected so that in protected form they have an overall positive oxidation, but can be cleaved intracellularly via cellular machinery (for example esterases) into an active form that has a variable oxidation state so that the probe is sensitive to changes to pH.

The pH independent fluorophore A allows for a ratiometric approach to be applied and provides an internal ratiometric standard. In a preferred embodiment, the pH independent fluorophore A is a cationic fluorophore. In a further preferred embodiment, the pH independent fluorophore A is a lipophilic fluorophore. In an especially preferred embodiment, the the pH independent fluorophore A is a cationic lipophilic fluorophore. By using a cationic and/or lipophilic flurophore, the probe can more easily pass through the plasma and mitochondrial membranes and the positive charge allows the probe to target the mitochondria. In a preferred embodiment, the pH independent fluorophore A is a mitochondrial targeting moiety.

In a preferred embodiment, the pH independent fluorophore A is selected from a fluorophore containing a positively charged N ion; a fluorophore derived from fluorones, polymethines, pyrenes or acridines; or a fluorophore derived from rosamines or cyanines. Particular non-limiting examples of pH independent fluorophore A includes: 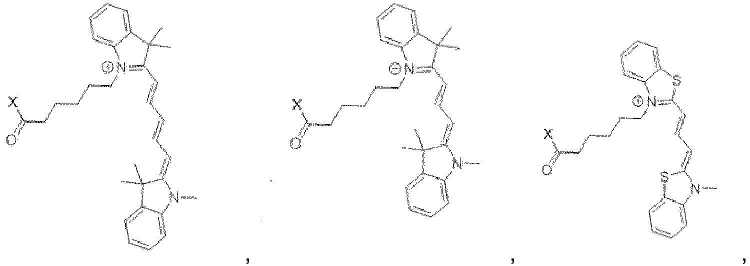 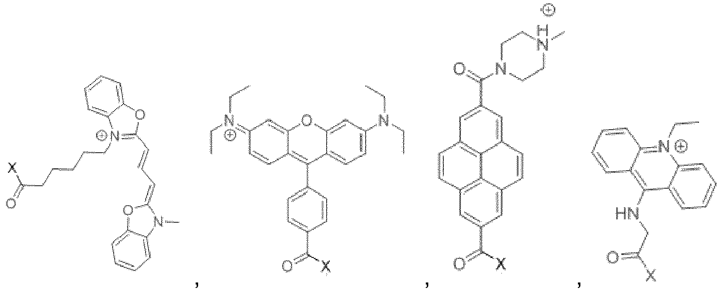 or 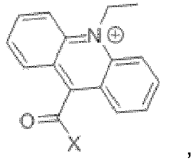 wherein X denotes the attachment site of L. In further embodiments, the attachment site X may be positioned in a different part of the fluorophore provided the position does not adversely affect the ability of the fluorophore to fluoresce in a pH independent manner.

Particularly preferred as pH independent fluorophore A is Cy5 and Cy3:

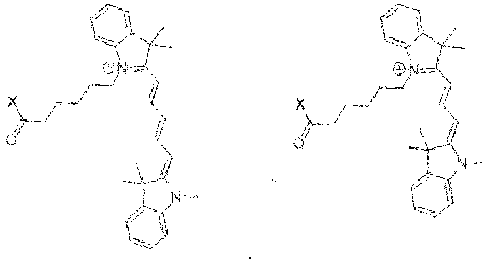

The attachment site X denoting the attachment site of L is shown at the carbonyl group at the end of the alkyl chain. Within the scope of the present invention it is envisaged that the attachment site could vary. Equally, groups that do not affect the fluorescence properties of the fluorophore can be modified, for example the carbonyl and alkyl groups and remain within the scope of the invention.

The pH dependent fluorophore B according to the present invention is capable of being protected such that the probe overall has an overall positive oxidation state. The fluorophore in inactive form must be capable of being converted into active form intracellularly. This may be achieved, for example, via esterases. In an embodiment, the pH dependent fluorophore B comprises oxygen groups which can be protected to lock or cage the molecule into a positive oxidation state. As such, in an embodiment, the pH dependent fluorophore B in inactive form comprises one or more protected oxygen(s).

Any suitable fluorophore can be used as pH dependent fluorophore B that allows for protection and cellular deprotection. Non-limiting examples of suitable fluorophores include fluoresceins or coumarins. In a preferred embodiment, the pH dependent fluorophore B is a fluorescein. In a further preferred embodiment, the pH dependent fluorophore B is a coumarin. In an embodiment, the pH dependent fluorophore B in inactive form may be selected from: 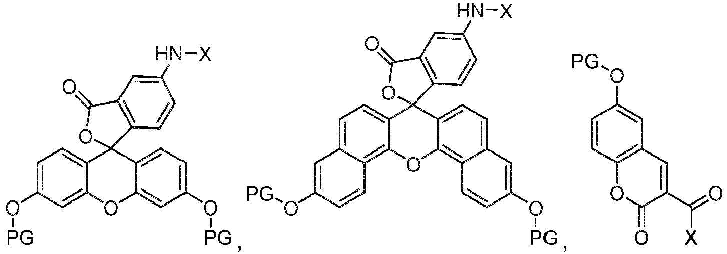 wherein X denotes the attachment site of L and PG denotes a protecting group. In a particularly preferred embodiment, the pH dependent fluorophore B is:

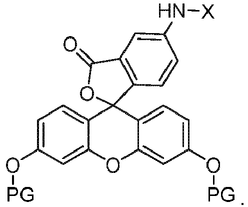

The attachment group X denotes the attachment site of L and is shown attached to the amine group. Variations to the attacment site are encompassed within the scope of the invention provided they do not adversely impact the ability of the fluorophore to function as a pH dependent fluorophore.

Particularly preferred mitochondrial pH probes include: 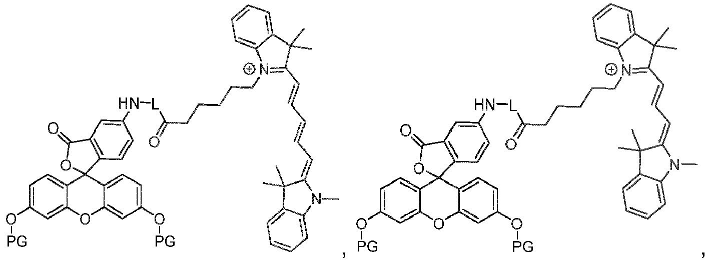 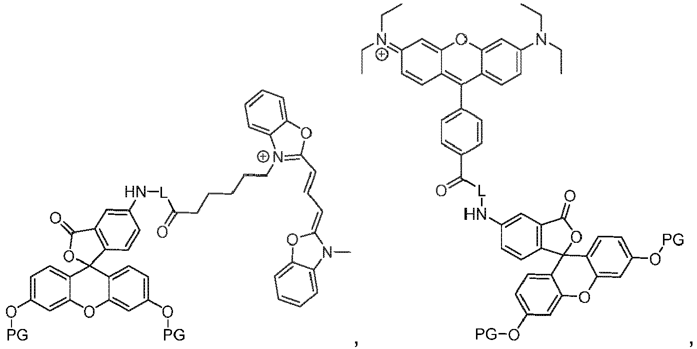 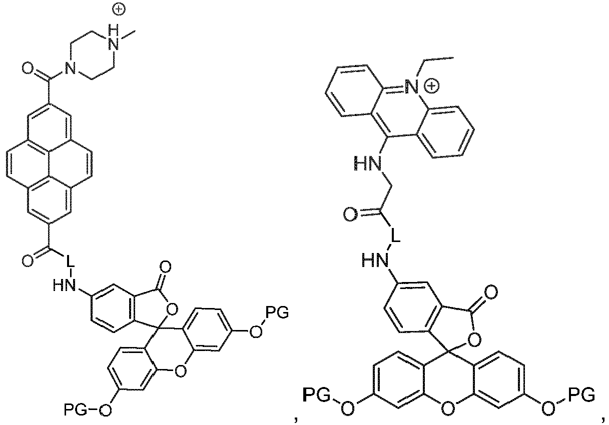 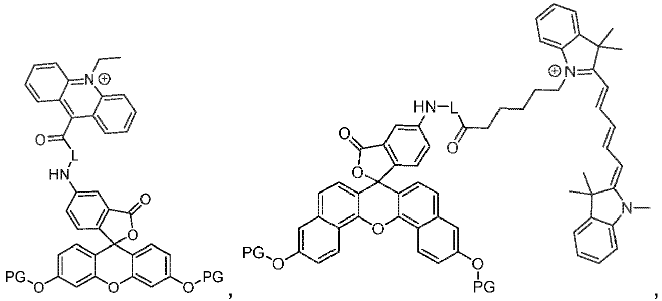 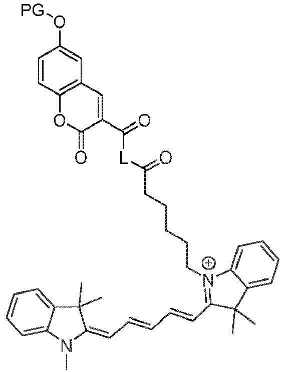 wherein PG and L are as defined herein.

An especially preferred probe is the flourescin-Cy5 and flourescin-Cy3 based probes:

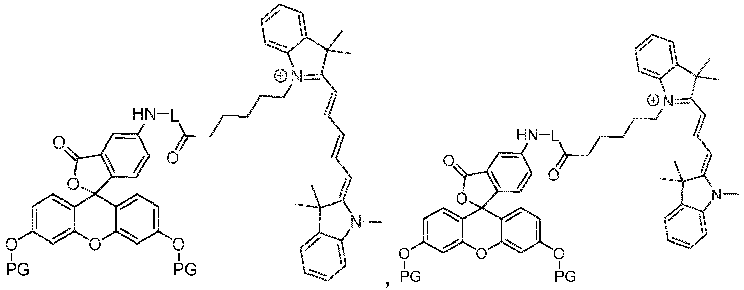

The linker L is selected from any suitable group that can covalently link the two fluorophores together without adversely affecting the fluorescence performance of either probe. In a preferred embodiment, the linker positioning and selection is designed to minimise steric hindrance for the rotation of the two fluorophores around the linker relative to each other, that may adversely affect the ability of the probe to travel through the cellular plasma membrane and mitochondrial membranes. In a yet further embodiment, the linker is a covalent bond between the two fluorophores.

In an embodiment, the linker L may be selected from one or more of: alkyl, alkenyl, alkynyl, cycloalkyl, aryl, heteroaryl, heterocyclic, heteroalkyl, carbonyl, alkylcarbonyl; and/or combinations thereof optionally joined directly or by an alkyl, carbonyl, aminocarbonyl, thiocarbonyl group or by a heteroatom.

In a further embodiment, the linker L may be of Formula II: L₁-(L₃)-L₂ (Formula II), wherein L₁ and L₂ are independently selected from alkyl, alkenyl, alkynyl, cycloalkyl, aryl, heteroaryl, heterocyclic, heteroalkyl, carbonyl, and alkylcarbonyl; and L₃ is a bond convalently linking L₁ and L₂ together; or L₃ is selected from heteroatom, alkyl, alkenyl, alkynyl, cycloalkyl, aryl, heteroaryl, heterocyclic, heteroalkyl, carbonyl, and alkylcarbonyl.

In a preferred embodiment, L₁ may be selected from: alkyl, heteroaryl, heterocyclic, heteroalkyl and alkylcarbonyl. In a further preferred embodiment L₁ may be selected from heteroaryl or alkylcarbonyl; more preferably 1,3,5-triazine or C₁₋ₙalkyl-1-carbonyl; wherein n is 2 to 12.

In a further preferred embodiment, L₂ may be selected from alkyl, heteroaryl, heterocyclic, heteroalkyl and alkylcarbonyl. In a yet further preferred embodiment, L₂ may be selected from alkyl, heterocyclic or heteroalkyl; more preferably piperazine, 1-amino-n-thio-C₁₋ₙalkyl, or 1,n-diamino-C₁₋ₙalkyl; wherein n is 2 to 12.

In a yet further preferred embodiment, L₃ may be absent or may be selected from: NH or S. In a particularly preferred embodiment, L₃ is absent.

Especially preferred linkers include:

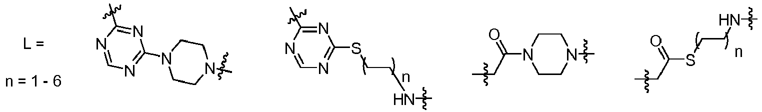

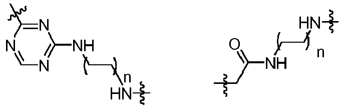

The mitochondrial pH probe according to the present invention in an inactive form is protected into an overall positive oxidation state. The protection is removed intracellularly to convert the probe into an active form with a variable oxidation state. As such, in an inactive form, the probe is protected by one or more protecting groups (PG). Any suitable protecting group can be used that is able to both achieve a positive oxidation state and also be removed intracellularly. Examples of possible protecting groups PG include acyl, propionyl, butyryl, isobutyryl, pivaloyl or benzoyl. An especially preferred protecting group is acyl.

Further possible preferred protecting groups include organic carbonate esters, thioesters and carbamates.

According to a preferred embodiment of the present invention, the mitochondrial pH probe in protected form comprises the molecule 5-FA-PIP-Cy5-DA (Formula III shown below). 5-FA-PIP-Cy5-DA comprises the fluorophore 5-FA linked to the fluorophore Cy5, via a PIP (piperazyl) linker. The 5-FA unit has been diacetylated, leading to the designation DA:

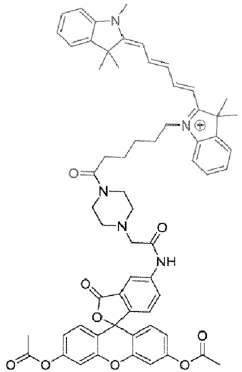

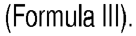

5-FA-PIP-Cy5-DA (and any other probes according to the present invention) is delivered to the into the cells in a non-active caged form, when the phenolic groups of fluorescein are acylated. This quenches the fluorescein's fluorescence and also removes the pH dependence of the probe. Once delivered to the cell, however, the acetyl groups are cleaved by the cellular (mostly mitochondrial) esterases and the probe is converted to the active form. This is shown schematically below which shows the mechanism of cellular deprotecting and conversion of the probe from a positive fixed inactive state into an active form which has pH dependent fluorescence.

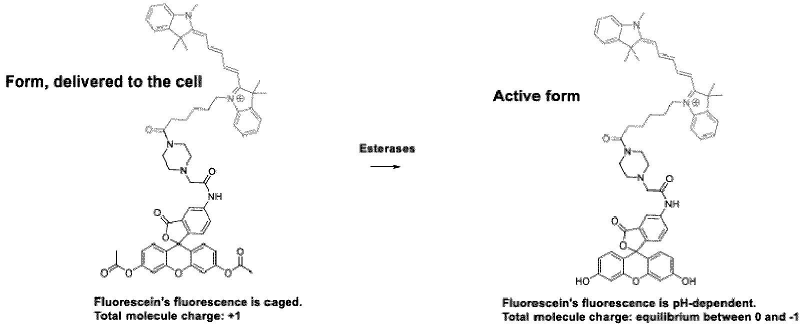

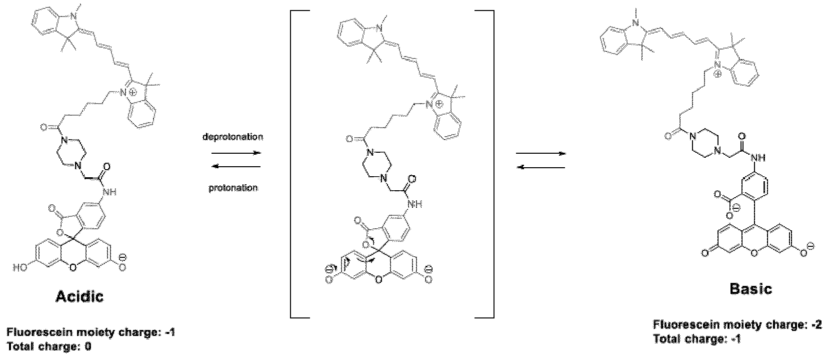

In the active form of the probe fluorescein's fluorescence is pH dependent due to the reversible protonation/deprotonation of its phenolic group, when in a more acidic environment the non-fluorescent closed monoanionic spiro-form of fluorescein is prevalent, while in more basic environment the equilibrium is shifted to the opened fluorescent dianionic form of fluorescein. The Cy5 fluorophore of the probe is always fluorescent and thus acts as the pH independent ratiometric internal standard.

The mitochondrial pH probes of the present invention can be used to measure mitochondrial pH. In an embodiment, there is provided a method of measuring mitochondrial pH comprising the steps of:
a) contacting a cell with a mitochondrial pH probe in an inactive form as defined herein;
b) allowing the probe to accumulate in the mitochondria of the cell, and to convert into an active form;
c) determining the ratio of fluorescence between the pH independent fluorophore A and the pH dependent fluorophore B; and
d) using this ratio to determine the mitochondrial pH.

When in inactive form, the probes of the present invention are configured to more easily pass through lipid membranes and to target the mitochondria. This leads to an accumulation of the probe in the mitochondria. As already discussed above, this accumulation is defined by the rate of influx into the cell and mitochondria and the rate of efflux by the cell's defence mechanisms to remove the probe. By ensuring rapid influx and effective mitochondrial targeting, the probe will accumulate in the mitochondria and influx will override efflux. The cell converts the probe into an active form, for example by using esterases. Esterases are present throughout the cell but because of the rapid accumulation of the probe within the mitochondria, conversion into active form is also predominantly occurring within the mitochondria leading to an accumulation of probe in active form within the mitochondria.

The advantageous properties of the probes of the present invention are such in some embodiments, sufficient accumulation of active probe in the mitochondria can take place within 15-20 minutes. This enables rapid mitochondrial pH measurements to take place. The sensitivity and update speed of the probe gives the ability to monitor real time changes to mitochondrial pH in response to any stimulus or condition being applied to the cell.

Once sufficient accumulation has taken place, it is possible to begin measuring the fluorescence of the mitochondrial pH probe. After initial incubation time with the medium, containing probe in the inactive form, the cells are washed with the medium, not containing the probe. All the accumulated probe inside the mitochondria of the cells is converted into active form almost instantaneously. It is retained inside the mitochondria due to its ability to be trapped because of the membrane impermeability of the active form. The cells are then ready to be analyzed by fluorescent light microscopy or flow cytometry. The ratiometric fluorescent signal is acquired and quantified. Other methods, including but not limited plate-reading bulk analysis, can be used to analyze the stained cells. Overall, the staining and washing procedures can be performed within 30 minutes, after which the cells are ready for analysis.

Prior to taking a pH measurement, it may be necessary to calibrate the probe to align the fluorescence ratiometric readings to a specific pH value. This may be undertaken by analysing the fluorescence ratio in the set of control experiments, including staining the control cell samples in the set of buffers with defined pH. These buffers must contain the protonophore agents, allowing the equilibration of the mitochondrial matrix pH with the pH of the surrounding buffer. Such agents might include:
carbonylcyanide-4-trifluoromethoxyphenylhydrazone (FCCP) as described in To, M. S. et al. Mitochondrial uncoupler FCCP activates proton conductance but does not block store-operated Ca2+ current in liver cells. Arch. Biochem. Biophys. 495, 152-158 (2010); and/or carbonylcyanide-3-chlorophenylhydrazone (CCCP) as described in Ruas, J. S. et al. Underestimation of the maximal capacity of the mitochondrial electron transport system in oligomycin-treated cells. PLoS One 11, 1-20 (2016); and/or other mitochondrial protonophore and/or ionophore and/or ion-exchanging agent, such as valinomycin, nigericin antimycin and others, for example Lou, P.-H. et al. Mitochondrial uncouplers with an extraordinary dynamic range. Biochem. J. 407, 129-140 (2007); Kadenbach, B. Intrinsic and extrinsic uncoupling of oxidative phosphorylation. Biochim. Biophys. Acta - Bioenerg. 1604, 77-94 (2003).

The cells initially are stained with the mitochondrial pH probe in the same conditions, as used in the main experiments. Then the medium, containing the probe, is washed away and substituted with the corresponding series of defined pH buffers, containing the means to equilibrate the mitochondrial pH and the buffer pH, preferably FCCP or CCCP. The series of control cells then analyzed after 15 minutes with the same method, as used for the main experiments, to quantify the ratiometric fluorescent signal, corresponding to the exact mitochondrial pH value, defined by the buffer pH in the case of the control experiment. The dependence of ratiometric signal over the pH value is then analytically approximated with the mathematical equation, preferably linear equation. This equation is later used to convert the value of ratiometric fluorescent signal, obtained in the main experiments, to the mitochondrial pH value, observed experimentally.

Through the ability to rapidly and sensitively measure pH changes within mitochondria, it is possible to evaluate how changes to the cell (for example the addition of a drug or drugs, changes in temperature or other external factors or the like) influence mitochondrial pH. It is equally possible to consider the reverse and investigate how changing mitochondrial pH can impact cellular activity. Non-limiting examples of the application of the mitochondrial pH probes of the present invention include
- Defining homeostatic mitochondrial pH values in various cell types;
- Defining changes in mitochondrial pH during the specific changes in particular cell types upon certain cellular activities, including but not limited proliferation, stress, apoptosis induction, quiescence, division and differentiation;
- Defining changes in mitochondrial pH upon pharmacological treatment;
- Defining changes in mitochondrial pH, characteristic for certain cell types upon onset of diseases, including but not limited cancer, diabetes and neurodegenerative diseases, or senescence;
- Defining changes in mitochondrial pH upon supplying various metabolic cues.

The probes of the present invention may be made by any suitable means. According to one embodiment, there is provided a method of manufacturing a dual fluorophore ratiometric mitochondrial pH probe of Formula I:

A-L-B (Formula I);

wherein:
A is a pH independent fluorophore;
B is a pH dependent fluorophore; and
L is a chemical linker, conjugating the pH independent fluorophore A to the pH dependent fluorophore B, or L is a covalent bond;
as defined herein, comprising protecting the pH dependent fluorophore B such that it is put into a positive oxidation state.

The invention will now be described by way of the following non-limiting examples.

### Examples

In the following examples the compound having the following formula III is referred to as 5-FA-PIP-Cy5-DA. 5-FA-PIP-Cy5-DA comprises the fluorophore5-FA (fluorescein) linked to the fluorophore Cy5, joined via a PIP (piperazyl) linker. The 5-FA unit has been diacetylated, leading to the designation DA.

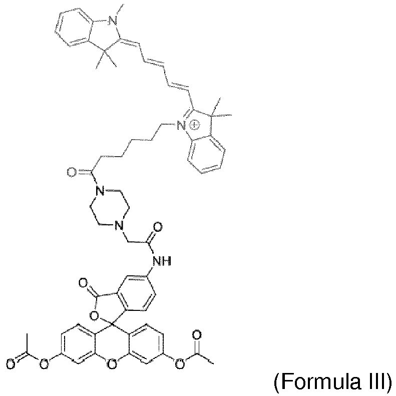

5-FA-PIP-Cy5-DA was synthesised from commercially available 5-aminofluoresin 17 according to the synthetic scheme below.

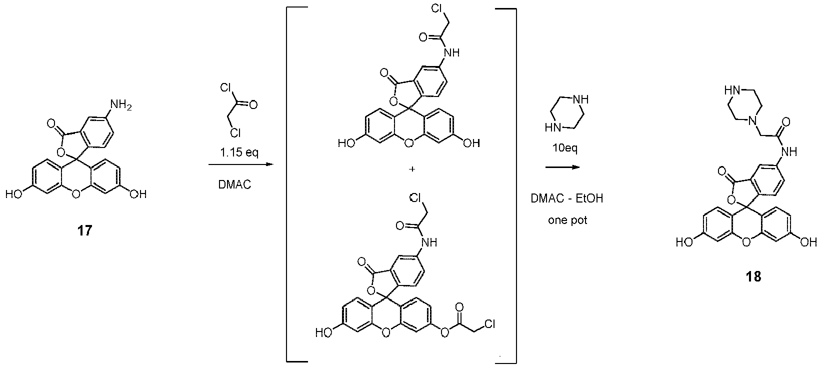

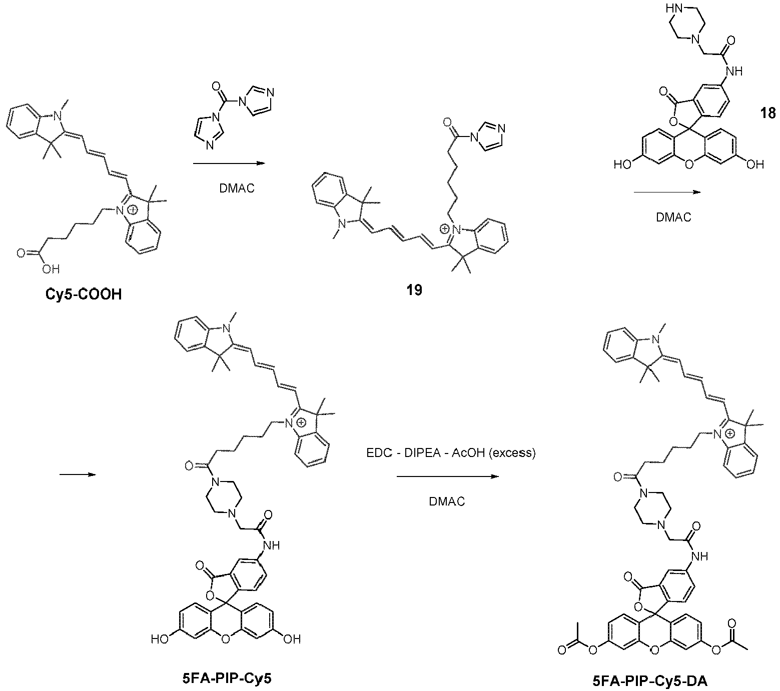

### Synthesis of 5-aminofluoresceinopiperazine 18

To a solution of 100 mg (0.288 mmol, 1 eq.) of 5-aminofluorescein 17 in 3 ml of DMAC a solution of 37 mg of chloroacetyl chloride (0.331 mmol, 1.15 eq.) in 1 ml of DMAC was added. The mixture was stirred for 10 min at room temperature and then a solution of 313 mg of piperazine (2.882 mmol, 10eq.) in 1 ml of ethanol was added. The mixture was stirred for 1 h. NMR spectra showed that the reaction was completed. The reaction mixture was then transferred to a falcon tube and 15 ml of diethyl ether was added to precipitate the crude product, which was separated by centrifugation. Next, 7 ml of ethanol was added to the separated precipitate and the resulting suspension sonicated for 2 minutes. The procedure was repeated 5 times and the purity of the product was controlled by NMR. Yield: 100 mg (81%) of 5-aminofluoresceinopiperazine 18.
¹H NMR (800 MHz, DMSO-d6) δ 10.17 (s, 1H), 8.36 (d, J = 1.7 Hz, 1H), 7.94 (dd, J = 8.3, 2.0 Hz, 1H), 7.20 (d, J = 8.3 Hz, 1H), 6.67 - 6.63 (m, 2H), 6.59 (d, J = 8.7 Hz, 2H), 6.53 (dd, J = 8.7, 2.3 Hz, 2H), 3.14 (s, 2H), 2.77 (t, J = 4.8 Hz, 4H), 2.47 - 2.42 (m, 4H).
HRMS (LC-MS) found: 474.1631, calculated for [M-H+]: 474.1660.

### Synthesis of 5FA-PIP-Cy5

5 mg of Cy5-COOH chloride (9.634 µmol, 1 eq.) was dissolved in 1 ml of DMAC in a 2 ml Eppendorf tube. To the resulting solution of 3.1 mg of carboxydiimidazole (19.268 µmol, 2 eq.) was added in 0.5 ml of DMAC. The mixture was stirred for 1 h. The completion of conversion of Cy5-acid to imidazolide 19 was controlled by NMR. Next, to the reaction mixture a solution of 5-aminofluoresceinopiperazine 18 (14.451 µmol, 1.5 eq.) in 0.5 ml of DMAC was added. The reaction mixture was stirred overnight at room temperature. After completion of the reaction (confirmed by NMR) the reaction mixture was diluted with 1 ml of chloroform and excess 5-aminofluoresceinopiperazine extracted 10 times with 0.5 ml of 0.5 M HCI, until the water layer was transparent. The remaining slurry of the chloroform layer was transferred to a 1.5 ml Eppendorf tube and the product was precipitated with 1 ml of diethyl ether. This precipitate was separated by centrifugation and the liquid layer was discarded. Next, to the remaining precipitate 0.3 ml of THF was added and the mixture sonicated for 2 minutes. The precipitate was separated by centrifugation again and the sonication-centrifugation was repeated 7 times. The precipitate was dried in the open Eppendorf tube in the darkness. The purity of the product was controlled by NMR. Yield: 9.7 mg (90%) of 5FA-PIP-Cy5, calculated for the dichloride form.
¹H NMR (500 MHz, DMSO-d6) δ 10.24 (s, 1H), 10.14 (s, 2H), 8.41 - 8.30 (m, 3H), 7.94 (s, 1H), 7.63 (t, J = 6.6 Hz, 2H), 7.42 (q, J = 7.8 Hz, 4H), 7.26 (t, J = 7.0 Hz, 3H), 6.69 (d, J = 2.0 Hz, 2H), 6.65 - 6.53 (m, 6H), 6.30 (dd, J = 24.6, 13.8 Hz, 2H), 4.12 (t, *J =* 6.8 Hz, 2H), 3.62 - 3.60 (m, 4H), 3.51 (s, 3H), 2.32 (s, 2H), 1.75-1.71 (m, 2H), 1.70 (s, 6H), 1.69 (s, 6H), 1.60-1.54 (m, 2H), 1.43-1.36 (m, 2H).
HRMS (LC-MS) found: 938.4008, calculated for [M-H+]: 938.4487.

### Synthesis of 5-FA-PIP-Cy5-DA

To the solution of 5-FA-PIP-Cy5 ((9.039 µmol, 1 eq.) in 1 ml of DMAC, a solution of 6.1 mg EDCI hydrochloride (24.118 µmol, 3eq.), 4.8 mg of AcOH (80.393 µmol, 10 eq.), and 3.1 mg of DIPEA (24.118 µmol, 3eq.) in 0.5 ml of DMAC was added. The mixture was stirred overnight at room temperature and the degree of acylation was controlled by NMR. Upon completion of acylation, the reaction mixture was transferred to 15 ml falcon tube and diluted with 2 ml of chloroform. The resulting mixture was extracted with 2 ml of 0.5 M HCI 5 times. The water layers were discarded and the remaining chloroform layer was transferred to a 2 ml Eppendorf tube and precipitated with 1 ml of diethyl ether to separate the product. The formed precipitate was separated by centrifugation. Next, to the precipitate 0.4 ml of THF was added and the mixture was sonicated for 2 minutes. To this mixture 1 ml of diethyl ether was added to crystallize back the dissolved product and the resulting precipitate was separated by centrifugation again. This procedure was repeated 5 times. The purity of the product was controlled by NMR. Yield: 8.2 mg (93%) of 5FA-PIP-Cy5-DA, calculated for the dichloride form.
¹H NMR (500 MHz, DMSO-*d*₆) δ 10.27 (s, 1H), 8.44 (d, *J =* 1.5 Hz, 1H), 8.33 (t, *J* = 13.1 Hz, 2H), 8.01 - 7.94 (m, 1H), 7.61 (t, *J* = 7.5 Hz, 2H), 7.44 - 7.36 (m, 4H), 7.34 (d, *J* = 8.4 Hz, 1H), 7.28 (d, *J* = 2.1 Hz, 2H), 7.26 - 7.22 (m, 2H), 6.95 (dd, *J* = 8.7, 2.1 Hz, 2H), 6.91 (d, *J* = 8.6 Hz, 2H), 6.56 (t, *J* = 12.3 Hz, 1H), 6.28 (dd, *J* = 25.5, 13.8 Hz, 2H), 4.10 (t, *J* = 7.2 Hz, 2H), 3.60 (s, *J* = 1.8 Hz, 3H), 3.54-3.45 (m, 4H), 3.23 (s, 2H), 2.33 - 2.30 (m, 2H), 2.29 (s, 6H), 1.71 - 1.73 (m, 2H), 1.68 (s, 6H), 1.67 (s, 6H), 1.55 (p, *J* = 7.3 Hz, 2H), 1.39 (p, *J* = 7.9 Hz, 2H).
¹³C NMR (201 MHz, DMSO-d₆) δ 173.75, 173.03, 170.84, 169.45, 169.34, 168.79, 154.50, 154.48, 152.52, 151.32, 147.12, 143.24, 142.55, 141.58, 141.49, 141.13, 129.65, 128.91, 128.83, 127.48, 126.72, 125.81, 125.21, 125.12, 124.97, 122.92, 122.77, 119.07, 116.71, 114.64, 111.56, 111.52, 110.94, 103.76, 103.54, 81.47, 61.89, 53.38, 52.88, 49.35, 49.33, 45.30, 43.77, 41.39, 32.39, 31.56, 27.67, 27.47, 27.29, 26.30, 25.00, 21.34.
HRMS (LC-MS) found: 1022.4530, calculated for [M-H+]: 1022.4699.

### Cell imaging

A2780 ovarian cancer cells were cultured in 24-well microscopy glass bottom plates in a CO2 incubator. Prior to imaging, the cells were stained with 2uM 5FA-PIP-Cy5-DA, and/or TMRM (20nM) for 30 min in bicarbonate-free DMEM medium to avoid extracellular pH fluctuations, then washed with the same medium (without 5FA-PIP-Cy5-DA) and imaged with confocal spinning disk microscopy at 60x magnification When TMRM was used, this dye was also present in the bicarbonate-free DMEM medium during the imaging of the cells. Separate fluorescent images in green (488/520 nm) and deep red (640/660 nm) spectral channels were acquired on Visitron W1 spinning disk confocal microscope. The florescence ratio was quantified using a specifically designed batch ImageJ plugin. This software thresholds the pH-independent Cy5 channel, quantifies the fluorescence intensity in the thresholded image areas, and uses the thresholded mask to quantify the intensity of pH-dependent 5Fluo channel. The intensity ratio is then used to estimate the mitochondrial pH of the cells. Calibration of the intensity ratio was achieved through the staining of A2780 cells in a series of PBS - 5% BSA buffers with defined pH, containing 20mM HEPES and 5uM FCCP, to equilibrate the mitochondrial pH with the pH of the buffer.

### Example 1 Active probe pKa and fluorescence study

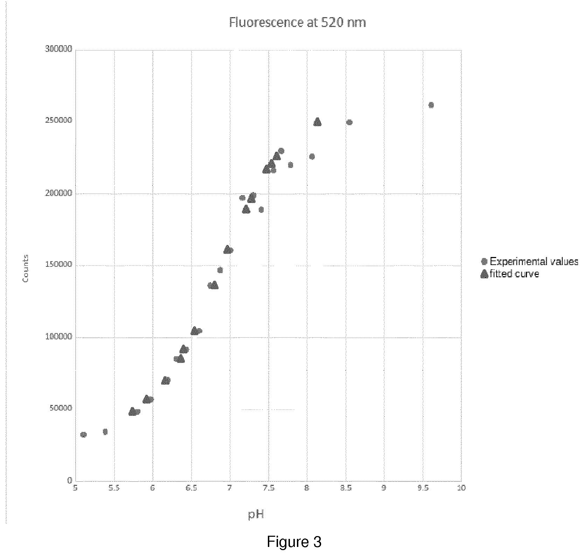

The pH dependence for fluorescence at 520 nm of 5FA-PIP-Cy5, the active form of 5FA-PIP-Cy5-DA was measured with 5FA-PIP-Cy5 dissolved in a PBS buffer with 5% BSA. (see Figure 3). From this study the pKa of the active form was estimated to be 6.88. This value is within expectable range for the fluorescein moiety containing electron-donor amide group and is within the physiologically relevant range of 6.5 - 8.5.

### Example 2 Testing the mitochondrial pH of live cells and the development of a calibration curve

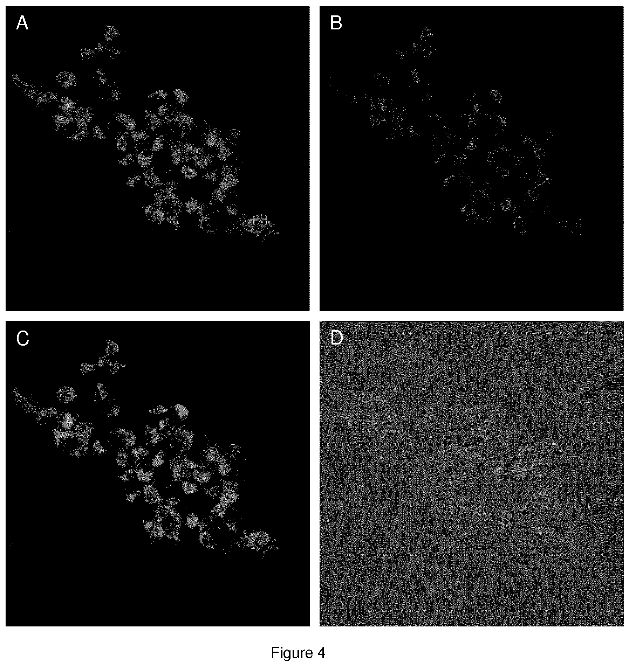

Typical pH variations in mitochondria of A2780 ovarian cancer cell line are represented at Figure 4. Typical staining procedure is described further. Cells were stained for 30 min with 2 µM 5FA-PIP-Cy5-DA solution, the probe was washed away with the bicarbonate-free DMEM medium, and the cells were imaged after 10 min with confocal spinning disk microscope. All images are maximum intensity projection. Figure 4A represents the 5FA-PIP-Cy5-DA pH-independent (Cy5) channel, shown in red on the figure. The mitochondrial staining on Figure 4A is very homogeneous. Figure 4B represents the 5FA-PIP-Cy5-DA pH-dependent (5Fluo) channel, shown in blue on the figure. The mitochondrial staining on Figure 4B is very heterogeneous, unlike the Figure 4A. Figure 4C represents the overlap image of Figure 4A and Figure 4B. Bluish hint Figure 4C corresponds to relatively high mitochondrial pH, reddish to relatively low mitochondrial pH. Figure 4D represents bright field image. On the Figure 4C it can be clearly seen, that the mitochondria in different A2780 cells are highly heterogeneous in the pH, which is reflected in the highly heterogeneous staining in pH dependent channel on Figure 4B.

The ability of the synthesised probe to distinguish mitochondrial pH changes in live cells was tested. This test was performed via the comparison of the fluorescein/Cy5 fluorescence ratio between control conditions and cells which had been treated with K⁺/H⁺ exchanging ionophore nigericin and a protonphore FCCP, which reduce mitochondrial pH by acidifying the mitochondrial matrix. FCCP and nigericin were commercially purchased. After the of A2780 ovarian cancer cells were stained as described above, the cells were washed with the bicarbonate-free DMEM medium, either containing DMSO vehicle for control sample or 300 nM FCCP or 2uM nigericin for the samples, where the mitochondrial pH was expected to be decreased. Confocal images were acquired for control, FCCP- and nigericin-treated cell samples, and the ratio between fluorescein and Cy5 channel fluorescence was quantified.

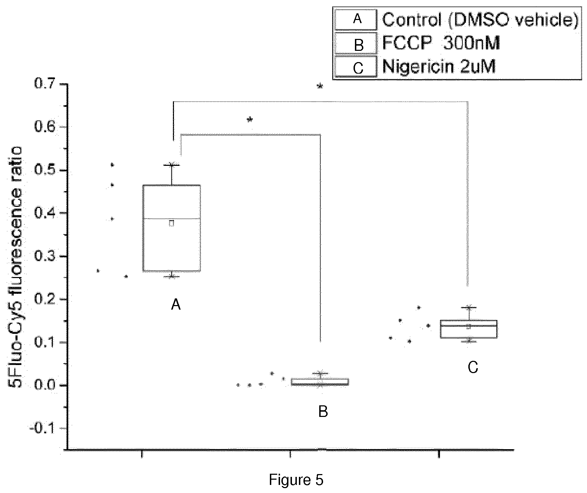

It can be seen from Figure 5 that treatment with both nigericin and FCCP resulted in a clear measurable decrease in the 5Fluo-Cy5 ratio. This validates that 5-FA-PIP-Cy5-DA taken into the cells and mitochondria, that it is converted into its active form 5FA-PIP-Cy5, and that the resultant probe is sensitive to mitochondrial pH changes.

Having validated that the probe is sensitive to mitochondrial pH changes, an experiment was performed to calibrate the probe.

The stained cells were treated with FCCP in high K⁺ buffers in a pH range of 6.73 to 8.36.

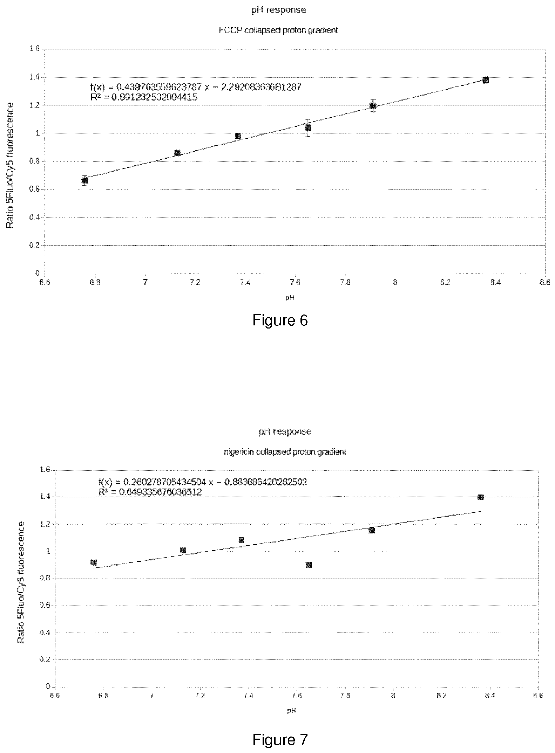

The results of the experiment can be seen in Figure 6. In FCCP treated cells within this pH range the fluorescein/Cy5 ratio showed a good linear response.

Treatment with FCCP alone caused leakage of the probe from the mitochondria into the cytoplasm, but the addition of 5% BSA to the calibration buffers greatly alleviated this problem, making the calibration with FCCP well-suited for mitochondrial pH calibration with the probe. In fact, treatment with FCCP caused full collapse of the mitochondrial pH gradient, ensuring that the pH to which the probe is exposed within the mitochondria is equal to the pH of the buffer.

Because the pH of the buffer can be directly measured, it was therefore possible to also know the exact cellular pH. As such, specific pH values can be attributed to the fluorescein/Cy5 ratios. FCCP treatment can therefore be used as a practical way to calibrate a cell for exact pH measurements.

The experiment was repeated using nigericin and the results can be seen in Figure 7. Again, a strong linear response is seen validating the reliability of the probe in measuring pH. However, no full gradient collapse was seen with nigericin, even at high concentrations. As such, the mitochondrial pH is not the same as the buffer and nigericin is not well suitable as an agent to prepare an accurate calibration curve.

### Example 2b

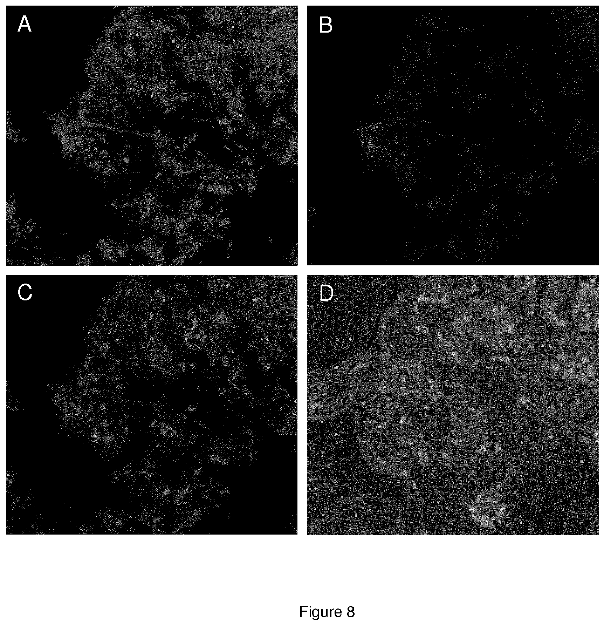

The selectivity of mitochondrial staining of 5FA-PIP-Cy5-DA was verified by co-staining with another well-known mitochondria targeting dye, tetramethylrhodamine methyl ester (TMRM). As 5FA-PIP-Cy5-DA is fluorescent in the deep red and green spectral regions, and TMRM is fluorescent in red spectral region, these dyes are spectrally compatible. A2780 cells were stained for 30 min with 2 µM 5FA-PIP-Cy5-DA and 20nM solution, the probe was washed away with the bicarbonate-free DMEM medium, containing 20nM TMRM, and the cells were imaged after 10 min with confocal spinning disk microscope. Figure 8 represents typical image of mitochondria, stained with both dyes. Figure 8A represents 5FA-PIP-Cy5-DA pH independent (Cy5) channel, shown in red. Figure 8B represents 5FA-PIP-Cy5-DA pH-dependent (5Fluo) channel, shown in blue. Figure 8C represents TMRM channel, shown in magenta. Figure 8D represents bright field image. Spinning disk confocal images of TMRM and 5FA-PIP-Cy5-DA costained A2780 cells were analyzed for pixel florescence intensity correlation between the Cy5 channel of 5FA-PIP-Cy5-DA and the TMRM channel (Figure 8). The Cy5 channel of 5FA-PIP-Cy5-DA is the most appropriate for co-localization studies, because it is pH-independent and therefore clearly reveals the localization of the 5FA-PIP-Cy5-DA molecule independent of the pH. The high degree of co-localization of the two dyes was confirmed by high values of Manders' coefficients (M1 = 0.94, M2 = 0.982) and Pearson's coefficient (r = 0.905). Importantly, it is consistently observed that very little cytoplasmic background is present during the staining with 5FA-PIP-Cy5-DA, demonstrating the highly selective mitochondrial staining by 5FA-PIP-Cy5-DA. Furthermore, high degree of colocalization with a well-known mitochondrial selective probe TMRM further confirms mitochondrial selectivity of 5FA-PIP-Cy5-DA.

### Example 2c

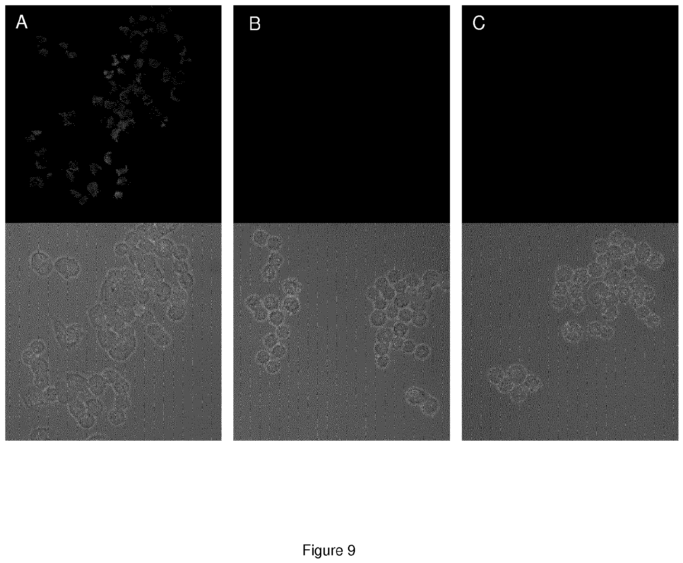

The advantage of the caging approach, ensuring the high lypophilicity and fixed positive oxidation state was tested by comparing the typical staining of the cells with the inactive form with the staining of the same cells in the same conditions directly with the active form. The result of such experiments are represented in Figure 9. Staining A2780 cells with 2uM of either 5FA-PIP-Cy5-DA or 5FA-PIP-Cy5 in identical conditions resulted in normal staining of mitochondria in the case of inactive form 5FA-PIP-Cy5-DA (Figure 9A) and absolutely no staining of mitochondria in the case of active form 5FA-PIP-Cy5 (Figure 9B), proving that the much more hydrophilic 5FA-PIP-Cy5 is not capable of permeating the cell membrane due to its non-cationic nature and higher hydrophilicity. In fact, there was no difference between the cells, stained with 5FA-PIP-Cy5 and non-stained control cells (Figure 9C).

### Example 3 Hematopoietic stem and progenitor cell studies

In order to demonstrate the applicability of the probes of the current invention in live cells, *in vitro* experiments were devised in which the mitochondrial pH differences in a series of live cells (LT-HSC, ST-HSC and MPP) was measured using through 5FA-PIP-Cy5-DA staining and fluorescence measurements. For LT-HSC cells, three different culture conditions (basal medium, DMOG-induced chemical hypoxia and IL-3, IL-6 - induced differentiation) were examined.

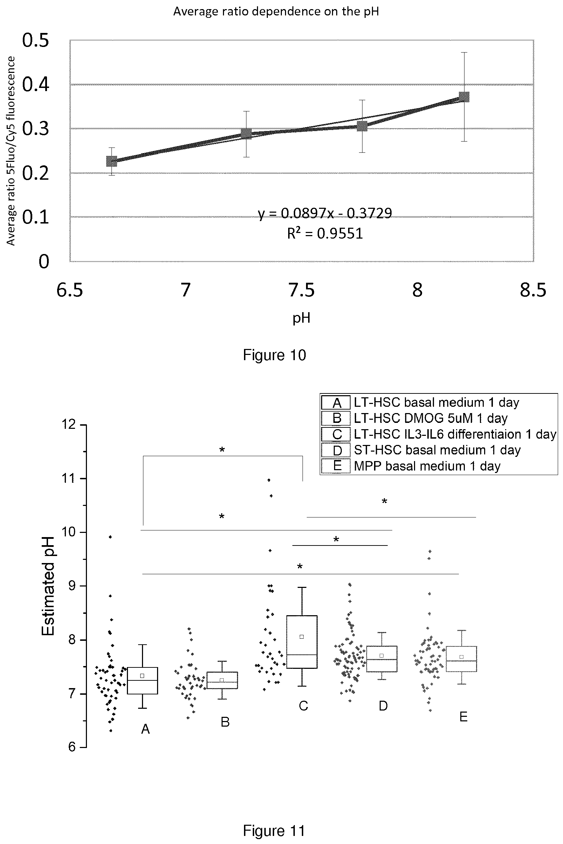

The calibration curve for the ratio of 5Fluoro/Cy5 fluorescence was obtained using FCCP and a PBS-5% BSA buffer with MPP cells in a manner analogous to the method described in Example 2 (see Figure 10).

Fresh cells, extracted from bone marrow of 8-12 week old male mice were stained on ice for surface cell markers and sorted by FACS, according to the following phenotypes: LT-HSC Lin- cKit+ Sca1+ CD150+ CD34- CD48-; ST-HSC Lin- cKit+ Sca1 + CD150+ CD34+; MPP Lin- cKit+ Sca1 + CD150- CD34+. The cells were cultured in Hematopoietic Stemline II (Sigma) medium with addition of SCF and Flt-3 cytokines. For appropriate experiments, additionally 5 uM DMOG or IL-3 20 ng/mL and IL-6 100ng/mL cytokines were added.

Prior to imaging, the cells were stained with the appropriate concentration of 5FA-PIP-Cy5-DA for 30 min in bicarbonate-free DMEM medium to avoid the extracellular pH fluctuation outside of the CO₂ incubator, then washed, transferred to poly-L-lysine coated 24-well microscopy glass bottom plates and imaged with confocal spinning disk microscopy at 60x magnification. Separate fluorescent images in green and deep red spectral channels were acquired. The florescence ratio was quantified by a specifically designed batch ImageJ plugin as described above.

LT-HSC showed a much lower mitochondrial pH level compared to ST-HSC and MPP (see Figure 11). LT-HSC, cultured in the basal medium, and LT-HSC, cultured in the presence of DMOG, had the same level of average mitochondrial pH, though DMOG-treated cells had less heterogeneity within the population, lacking the individual cells with high mitochondrial pH. Significantly, IL-3, IL-6 - treated LT-HSC had the highest overall average mitochondrial pH value, significantly above LT-HSC, both when cultured in basal medium, and with addition of DMOG, and ST-HSC and MPP.

This demonstrated that 5FA-PIP-Cy5-DA performs well as a probe for mitochondrial pH. The probe was able to demonstrate that differentiation induction in LT-HSC causes the burst in mitochondrial pH to a level not observed even in highly active, proliferative ST-HSC and MPP cells. Furthermore, through the use of 5FA-PIP-Cy5-DA, significant differences in mitochondrial pH were observed through the range of conditions, favouring different stem cell fate outcomes, and demonstrating that mitochondrial pH alteration is associated with hematopoietic stem cell fate determination as a result.

### Example 4: Anti-cancer drug treatment studies

*In vitro* studies were performed in which 5FA-PIP-Cy5-DA staining could be used to distinguish between direct drug interference with mitochondrial metabolism and general cytotoxicity from anti-cancer drugs RAPTA-T and cisplatin.

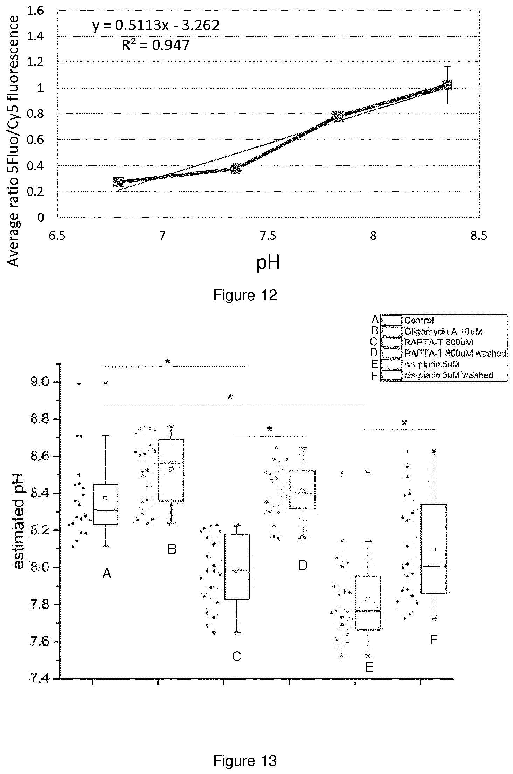

The calibration curve for the ratio of 5Fluoro/Cy5 fluorescence was obtained using FCCP and a PBS-5% BSA buffer with A2780 cells in a manner analogous to the method described in Example 2 (see Figure 12).

The quantifying of mitochondrial pH in the presence of the drug and after the drug was washed away allowed the distinction between interference with mitochondrial metabolism and general cytotoxicity. If the effect of the drug was primarily to affect mitochondrial metabolism it was expected that the mitochondrial pH would quickly return to the non-treated cell level following removal of the drug, providing the drug had no other significant cytotoxic effects. Conversely, if the effect of the drug were not reversed by the washing step, an observed alteration of mitochondrial pH could be interpreted at least partially as a downstream consequence of the general cytotoxicity of the drug.

A2780 cells were cultured in 24-well microscopy glass bottom plates in RPMI medium, containing Glutamax with 10% FBS addition. The cells were treated with appropriate 800uM RAPTA-T or 5uM cisplatin. Prior to the imaging the cells were stained with 2uM 5FA-PIP-Cy5-DA for 30 min in a bicarbonate-free DMEM medium to avoid the extracellular pH fluctuation outside of the CO₂ incubator, then washed and imaged with confocal spinning disk microscopy at 60x magnification. Cells were treated with either drug for one day and mitochondrial pH was quantified using 5FA-PIP-Cy5-DA in the presence of the drug and after the drug was washed away. For some cells the drugs were washed away prior to staining with 5FA-PIP-Cy5-DA. If the drug was not washed away prior to staining, it remained present in the medium through the whole imaging time. Separate fluorescent images in green and deep red spectral channels were acquired. The florescence ratio was quantified by a specifically designed batch ImageJ plugin as described above. Statistical analysis was performed using 1-way ANOVA with Bonferroni-Holm post-hoc analysis.

The results are shown in Figure 13 which clearly showed that cisplatin and RAPTA-T both drastically decreased mitochondrial pH in A2780 cells in comparison to non-treated cells. The introduction of RAPTA-T saw the pH drop to 8.0 compared to pH 8.4 seen with the control cells. Upon washing, the value rose again to pH 8.4, fully restoring the mitochondrial pH. By comparison, cisplatin dropped the pH to an average of 7.8, which increased to 8.0 after washing, with mitochondrial pH only partially restored by the washing step. This indicated that RAPTA-T has a much more direct impact on mitochondrial metabolism in comparison to cisplatin.

Again, it can be seen that the use of 5FA-PIP-Cy5-DA, enabled a rapid and direct measurement of mitochondrial pH allowing real time measurements of the effects of drugs on mitochondrial pH and to distinguish the differences of mechanisms of actions behind the alteration of mitochondrial pH by the anti-cancer drug treatment.

Figure 13 also demonstrates the use of 5FA-PIP-Cy5-DA to assess the functional state of mitochondrial ATP synthase of A2780 cells by measuring mitochondrial pH in the presence and absence of ATP synthase inhibitor oligomycin. It can be seen that the addition of 10µm oligomycin caused slight increase in mitochondrial pH from 8.4 to around 8.5.

Oligomycin inhibits proton conductance by ATP synthase, removing its contribution to the proton gradient. If mitochondrial ATP synthase acts in the normal mode, inhibition with oligomycin would increase mitochondrial pH, while in the reversed mode it would decrease mitochondrial pH. Finally, in the case of pre-existing natural inhibition of ATP synthase, inhibition with oligomycin would not noticeably change mitochondrial pH.

It can be seen from figure 13 that oligomycin caused a noticeable increase in mitochondrial pH demonstrating that the mitochondrial ATP synthase in the tested cells was acting in the normal mode. This suggests that the cytotoxic effects of the tested anticancer drugs in these cells were related to the disruption of mitochondrial oxidative phosphorylation: as during the normal functional state of mitochondrial ATP synthase, alkalinisation of mitochondrial matrix is achieved through the activity of respiratory chain, the following drug-induced drop in mitochondrial pH can be attributed to the disruption of this process.

In conclusion, these experiments demonstrate the potential and wide range of applications for 5-FA-PIP-Cy5-DA. The experimental data shows that due to the simplicity of the experiments, the impact on mitochondrial metabolism can be reliably and quickly assessed in real time. The data shows that 5-FA-PIP-Cy5-DA can be used to determine the specificity of mechanism of action of the various drugs which purport to have an effect on mitochondrial metabolism.

## Claims

1. A dual fluorophore ratiometric mitochondrial pH probe of Formula I:
A-L-B (Formula I);
wherein:
A is a pH independent fluorophore;
B is a pH dependent fluorophore; and
L is a chemical linker, conjugating the pH independent fluorophore A to the pH dependent fluorophore B, or L is a covalent bond;
wherein in an inactive form, the probe is protected in an overall positive oxidation state; and wherein the probe may be deprotected intracellularly into an active form with a variable oxidation state.

2. The mitochondrial pH probe of claim 1, wherein in the inactive form, the probe has an oxidation state of +1.

3. The mitochondrial pH probe of claim 1 or claim 2, wherein in the active form, the probe has a variable oxidation state of between 0 and -2, preferably between 0 and -1 or between -1 and -2.

4. The mitochondrial pH probe of any preceding claim, wherein the pH independent fluorophore A is selected from:
a. a fluorophore containing a positively charged N ion;
b. a fluorophore derived from fluorones, polymethines, pyrenes or acridines; or
c. a fluorophore derived from rosamines or cyanines;
wherein the pH independent fluorophore A is preferably selected from: 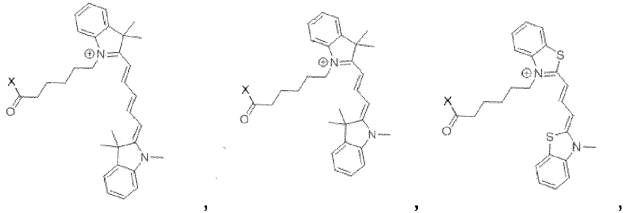 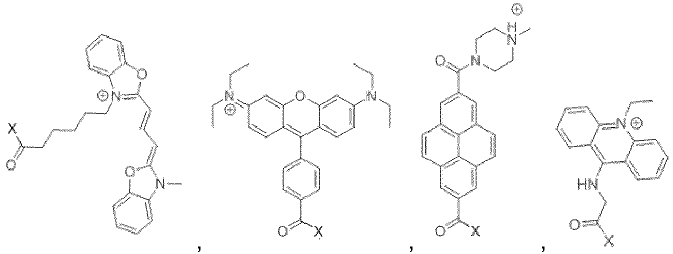 or 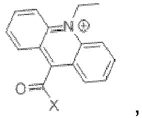 wherein X denotes the attachment site of L;
most preferably wherein the pH independent fluorophore A is Cy5: 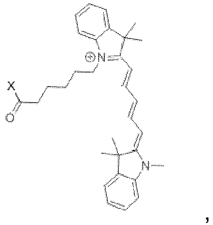 wherein X denotes the attachment site of L.

5. The mitochondrial pH probe of any preceding claim, wherein the pH dependent fluorophore B in inactive form is a fluorophore comprising one or more protected oxygen(s).

6. The mitochondrial pH probe of any preceding claim, wherein the pH dependent fluorophore B is selected from fluoresceins or coumarins;
preferably wherein the pH dependent fluorophore B in inactive form is selected from: 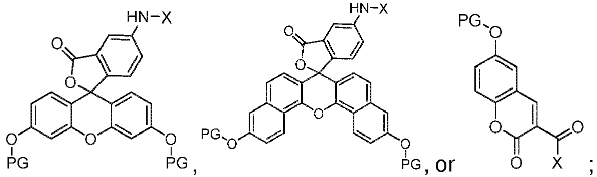 more preferably wherein the pH dependent fluorophore B in inactive form is: 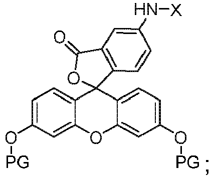 wherein X denotes the attachment site of L and PG denotes a protecting group.

7. The mitochondrial pH probe of claim 1, wherein the probe is selected from: 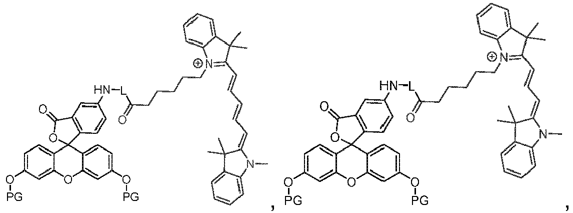 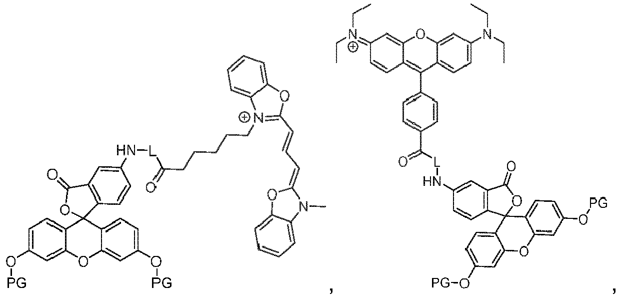 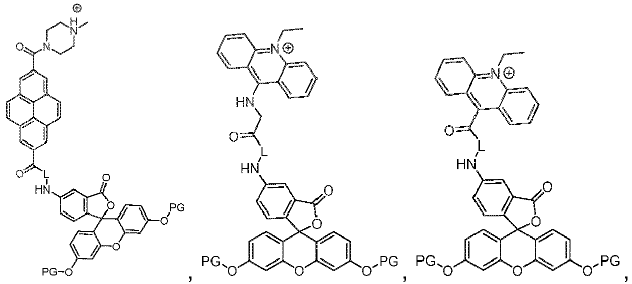 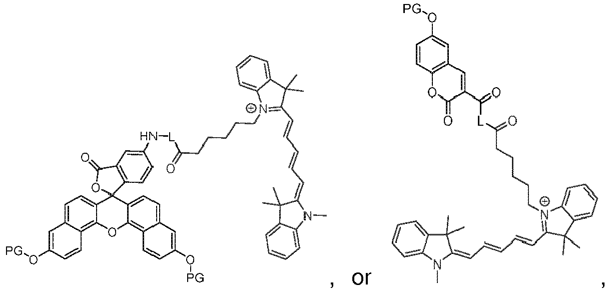 wherein PG is a protecting group;
most preferably wherein the probe is:

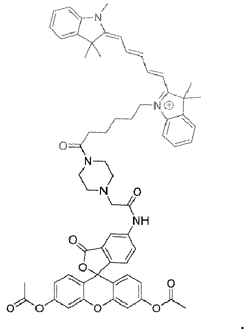

8. The mitochondrial pH probe of any preceding claim, wherein L is a linker selected from:
alkyl, alkenyl, alkynyl, cycloalkyl, aryl, heteroaryl, heterocyclic, heteroalkyl, carbonyl, alkylcarbonyl; and/or
combinations of the above, optionally joined directly or by an alkyl, carbonyl, aminocarbonyl, thiocarbonyl group or by a heteroatom.

9. The mitochondrial pH probe according to claim 12, wherein L is of formula II:
L₁-(L₃)-L₂ (Formula II)
wherein L₁ and L₂ are independently selected from alkyl, alkenyl, alkynyl, cycloalkyl, aryl, heteroaryl, heterocyclic, heteroalkyl, carbonyl, and alkylcarbonyl; and
L₃ is a bond convalently linking L₁ and L₂ together; or L₃ is selected from heteroatom, alkyl, alkenyl, alkynyl, cycloalkyl, aryl, heteroaryl, heterocyclic, heteroalkyl, carbonyl, and alkylcarbonyl;
wherein L₁ may optionally be is selected from: alkyl, heteroaryl, heterocyclic, heteroalkyl and alkylcarbonyl, preferably heteroaryl or alkylcarbonyl; more preferably 1,3,5-triazine or C₁₋ₙalkyl-1-carbonyl; wherein n is 2 to 12; and/or
wherein L₂ may optionally be selected from: alkyl, heteroaryl, heterocyclic, heteroalkyl and alkylcarbonyl, preferably, alkyl, heterocyclic or heteroalkyl; more preferably piperazine, 1-amino-n-thio-C₁₋ₙalkyl, or 1,n-diamino-C₁₋ₙalkyl; wherein n is 2 to 12; and/or
wherein L₃ may optionally be selected from: S, NH, or is not present;
preferably, wherein the linker L is selected from: 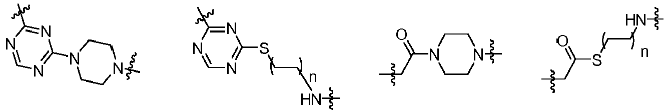 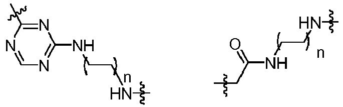
wherein n = 1-6.

10. The mitochondrial pH probe of any preceding claim, wherein the protecting group PG is selected from acyl, propionyl, butyryl, isobutyryl, pivaloyl or benzoyl; preferably acyl.

11. A method of measuring mitochondrial pH comprising the steps of:
a) contacting a cell with a mitochondrial pH probe in an inactive form as claimed in any one of claims 1 to 10;
b) allowing the probe to accumulate in the mitochondria of the cell, and to convert into an active form;
c) determining the ratio of fluorescence between the pH independent fluorophore A and the pH dependent fluorophore B; and
d) using this ratio to determine the mitochondrial pH;
wherein step a) may optionally comprise contacting the mitochondrial pH probe to a cell *in vivo* within a living organism or *in vitro,* for example within a 3D-cell culture model; and/or
wherein in step b) the probe may optionally accumulate in the mitochondria within 15 minutes in sufficient quantity to determine mitochondrial pH.

12. The method of claim 11, wherein in step b) the probe is converted into an active form by esterases.

13. The method of claim 11 or claim 12, wherein the probe is calibrated using FCCP or other protonophore or similar agent, allowing equilibration of the mitochondrial pH with the pH of the buffer.

14. A method of measuring the impact of drugs or other conditions on the mitochondrial pH comprising applying the drug or other condition to a cell and measuring the change in mitochondrial pH using a dual fluorophore ratiometric mitochondrial pH probe as claimed in any one of claims 1 to 10.

15. A method of manufacturing a dual fluorophore ratiometric mitochondrial pH probe of Formula I:
A-L-B (Formula I);
wherein:
A is a pH independent fluorophore;
B is a pH dependent fluorophore; and
L is a chemical linker, conjugating the pH independent fluorophore A to
the pH dependent fluorophore B, or L is a covalent bond;
as claimed in any one of claims 1 to 10, comprising protecting the pH dependent fluorophore B such that the mitochondrial pH probe of Formula I is put into a positive oxidation state.
